# EUROPEAN PATENT SPECIFICATION

(11) **EP 1 896 045 B1**
(45) Date of publication and mention of the grant of the patent: **17.08.2016**
(21) Application number: 06779752.2
(22) Date of filing: 22.06.2006
(51) Int. Cl.: A01N 65/00

(54) **COMPOSITION COMPRISING BRASSICACEA SEEDS MEAL AND THEIR USE AS BIOPESTICIDES IN PLANTS**
ZUSAMMENSETZUNG DIE BRASSICACEA KERNMEHL ENTHALTET UND IHRE VERWENDUNG ALS BIOPESTIZIDEN IN PFLANZEN
COMPOSITION COMPRENANT DE LA FARINE DE GRAINES DE BRASSICACEAE ET LEUR UTILISATION COMME BIOPESTICIDES CHEZ LES PLANTES

(30) Priority: 22.06.2005 IT BO20050416
(43) Date of publication of application: 12.03.2008
(73) Proprietor: Rongai, Domenico, 64020 Roseto Degli Abruzzi (IT); Cerato, Claudio, 40013 Castelmaggiore (IT); Lazzeri, Luca, 50129 Firenze (IT); Palmieri, Sandro, 40120 Bologna (IT)
(72) Inventor: Rongai, Domenico, 64020 Roseto Degli Abruzzi (IT); Cerato, Claudio, 40013 Castelmaggiore (IT); Lazzeri, Luca, 50129 Firenze (IT); Palmieri, Sandro, 40120 Bologna (IT)
(74) Representative: Mangini, Simone
(86) International application number: PCT/IB2006/001700
(87) International publication number: WO 2006/136933

(56) References cited:
- WO-A-2006/065736
- US-A- 5 866 762
- US-B1- 6 545 043
- SMOLINSKA U ET AL: "Toxicity of glucosinolate degradation products from Brassica napus seed meal toward Aphanomyces euteiches f. sp. pisi" PHYTOPATHOLOGY, vol. 87, no. 1, 1997, pages 77-82, XP008070558 ISSN: 0031-949X
- DATABASE BIOSIS [Online] BIOSCIENCES INFORMATION SERVICE, PHILADELPHIA, PA, US; 1997, BOREK VLADIMIR ET AL: "Toxicity of rapeseed meal and methyl isothiocyanate to larvae of the black vine weevil (Coleoptera: Curculionidae)" XP002265107 Database accession no. PREV199799422007 & JOURNAL OF ECONOMIC ENTOMOLOGY, vol. 90, no. 1, 1997, pages 109-112, ISSN: 0022-0493
- MOMEN F M ET AL: "A comparative study of the effect of some mineral and plant oils on two predacious mites of the family Phytoseiidae (Acari: Phytoseiidae)." ACTA PHYTOPATHOLOGICA ET ENTOMOLOGICA HUNGARICA, vol. 38, no. 3-4, 2003, pages 377-384, XP008070726 ISSN: 0238-1249
- MARTIN BEGONA ET AL: "Effects of various oils on survival of Myzus persicae Sulzer and its transmission of cucumber mosaic virus on pepper" JOURNAL OF HORTICULTURAL SCIENCE & BIOTECHNOLOGY, vol. 79, no. 6, November 2004 (2004-11), pages 855-858, XP008070761 ISSN: 1462-0316
- MARI M ET AL: "In vitro activity of glucosinolate-derived isothiocyanates against postharvest fruit pathogens" ANNALS OF APPLIED BIOLOGY, vol. 123, no. 1, 1993, pages 155-164, XP001208100 ISSN: 0003-4746
- MANICI LUISA M ET AL: "Suppressive activity of some glucosinolate enzyme degradation products on Pythium irregulare and Rhizoctonia solani in sterile soil" PEST MANAGEMENT SCIENCE, vol. 56, no. 10, October 2000 (2000-10), pages 921-926, XP002405446 ISSN: 1526-498X
- MANICI LUISA M ET AL: "In vitro fungitoxic activity of some glucosinolates and their enzyme-derived products toward plant pathogenic fungi" JOURNAL OF AGRICULTURAL AND FOOD CHEMISTRY, vol. 45, no. 7, 1997, pages 2768-2773, XP008070663 ISSN: 0021-8561
- MARTIN-LOPEZ BEGONA ET AL: "Use of oils combined with low doses of insecticide for the control of Myzus persicae and PVY epidemics" PEST MANAGEMENT SCIENCE, vol. 62, no. 4, April 2006 (2006-04), pages 372-378, XP002405447 ISSN: 1526-498X
- BERTRAND MATTHÄUS ET AL: 'Glucosinolates in Members of the Family Brassicaceae: Separation and Identification by LC/ESI-MS-MS' JOURNAL OF AGRICULTURAL AND FOOD CHEMISTRY vol. 48, no. 6, 01 June 2000, pages 2234 - 2239, XP055213448 DOI: 10.1021/jf991306w ISSN: 0021-8561

## Description

### TECHNICAL FIELD

The present invention refers to the control of infestants and infectants and refers to a composition for the treatment and/or the prevention of attacks by biological agents, in particular to prevent or eliminate bacterial infections, mycoses, infestations or attacks by insects against plants and vegetables in general, organic objects such as wood, its by-products and the like. The composition comprises a meal of Brassicaceae seeds mixed with at least one oil or fat of vegetable, animal, mineral or synthetic origin, to form an emulsified mixture in water

### BACKGROUND ART

In the last 50 years, plant defence has required an increasing use of insecticides and synthesis treatments, very often providing only partial control of some phytophages due to the onset of resistant biotypes.

The repeated and continuous use of a pest control product is in fact due to a selective pressure in favour of the resistant variation to the same product (Sauphanor et al., 1996 - Phytoma 482, 16-21) and in some case the use of the same insecticide has even led to an increase in the reproductive capacity of the phytophage. Rongai et al. (1998 - Potato Research 41, 29-37) noted that in various experimental fields, starting in 1982, Aphis gossypii gradually increased over the years and that the traditional pirimicarb-based treatments were not only unable to limit the infestation, but actually caused a significant increase in the reproductive capacity of the treated aphids.

This phenomenon required an increase in the number of treatments with harmful consequences for the environment, consumers and operators in the sector, with symptoms ranging from acute forms of poisoning, such as those caused by phosphoric esters, to medium and long-term effects typical of dithiocarbamates and even to cancerogenous effects. Numerous strategies have been employed to reduce the use of synthesis insecticides, including:
1) specific distribution of treatment, in order to protect plants with as few interventions as possible (guided control);
2) supplementation of chemical control with other means (physical and agronomical) for plant defence (integrated control);
3) replacement of chemical means with agents such as viruses, bacteria, fungi, mites, nematodes and insects (biological control); 4) use of non-synthesis plant-origin products (control with natural products).

Smolinska U. et al ("Toxicity Of Glucosinolate Degradation Products From Brassica Napus Seed Meal Toward Aphanomyces Euteiches F. Sp. Pisi", PHYTOPATHOLOGY, Vol:87, Nr:1, Page(s):77 - 82, ISSN 0031-949X) discloses that *Brassica* tissues are potentially useful in the control of *Aphanomyces* root rot of peas (*Pisum sativum*)*,* but identity of the responsible compounds and specific impacts of those compounds on the pathogen's infection potential remain uncertain.

Borek Vladimir et al ("Toxicity of rapeseed meal and methyl isothiocyanate to larvae of the black vine weevil (Coleoptera: Curculionidae)" Journal of Economic Entomology, Volume 90, Number 1, February 1997, pp. 109-112(4), ISSN: 0022-0493) discloses that soil amendments of Brassica spp. tissues display toxic effects to a number of soil organisms, including insects. However, application rates necessary to obtain effective insect suppression have not been determined.

Martin Begona et al ("Effects of various oils on survival of Myzus persicae Sulzer and its transmission of cucumber mosaic virus on pepper", JOURNAL OF HORTICULTURAL SCIENCE AND BIOTECHNOLOGY, 20041101 Headley Brothers, Ashford, GB - ISSN 1462-0316) discloses the use of mineral oils for controlling non-persistently transmitted viruses by aphid vectors, but mineral oils cause a number of problems including significant phytotoxicity. Three series of assays were carried out to compare a mineral oil, a fish oil and four vegetable oils for: a) insecticidal effects on the aphid Myzus persicae; b) inoculation of cucumber mosaic virus (CMV) by M. persicae to oil-sprayed pepper plants; and c) acquisition of CMV by M. persicae from oil-sprayed pepper plants. The vegetable oils tested were raw and refined rapeseed oil, and raw and refined soya oil. The oil showing the strongest insecticidal activity was raw soya oil. Refined soya oil, both rapeseed oils and fish oil also showed appreciable insecticidal activity.

### DISCLOSURE OF THE INVENTION

One object of the present invention, in the control of infestants, infectants and harmful biological agents, is to replace synthesis active ingredients with a composition of natural products through a formulation of oil or other modulator means with meal of Brassicaceae seeds that have a cytotoxic activity against some parasites and pathogens of plants, wooden objects or other materials subject to infestation. The composition comprises a meal of Brassicaceae seeds mixed with at least one oil or fat of vegetable, animal, mineral or synthetic origin, to form an emulsified mixture in water. The product that can be marketed in one- or more-litre bottles without any special precautions since (especially if vegetable oil is used in the composition) it presents little or no toxicity for man in this stage.

The characteristics of the present invention are underlined in the following with reference to the attached drawings, in which:
- Figure 1 shows mortality (in % with respect to the control). The histograms with different letters are statistically different (P < 0.05). 1=mineral oil in an aqueous solution; 2=meal with sinigrin in an aqueous solution; 3=mineral oil + meal with sinigrin in an aqueous solution.
- Figure 2 shows mortality (in % with respect to the control). The histograms with different letters are statistically different (P < 0.05). 1=vegetable oil in an aqueous solution; 2=meal with sinigrin in an aqueous solution; 3=vegetable oil + meal with sinigrin in an aqueous solution.
- Figure 3 shows mortality (in percentage) of various glucosinolates compared to the mortality due to the action of mineral oil. B. carinata meal used in water without oil. In this case the values were corrected by untreated control. The histograms with different letters are statistically different (P < 0.05). 1=Mineral oil+B. carinata meal; 2=Mineral oil+ H. annuus meal; 3=Mineral oil+ B. verna metal; 4=Mineral oil+ E. sativa meal.
- Figure 4 shows mortality (in percentage) of various meals. The values were corrected with respect to the control with just oil. The data in plot 3 were corrected with the untreated control. The histograms with different letters were statistically different (P < 0.05). 1=mineral oil+ B. carinata meal; 2=mineral oil + sunflower meal; 3= B. carinata meal directly in water; 4=mineral oil + deactivated B. carinata meal.
- Figure 5 shows mortality (in percentage of the control) of 3^{rd} generation red scale insects. The histograms with different letters are statistically different (P < 0.05). 1= vegetable oil + B. carinata meal; 2= vegetable oil + synthesis isothiocyanate.
- Figure 6 shows total number of scale insects present on 100 fruits. The histograms with different letters are statisfically different (P < 0.05). 1=untreated control; 2= mineral oil + B. carinata meal; 3= vegetable oil + B. carinata meal.
- Figure 7 shows number of hatched eggs (in percentage) of European red mite (Panonychus ulmi). The histograms with different letters are statistically different (P < 0,05). 1= control; 2= vegetable oil; 3= vegetable oil + B. carinata meal; 4= mineral oil + B. carinata meal; 5= commercial acaricide.
- Figure 8 shows leaf tissue (% of the control) of beet plants infected by mildew *Erysiphe betae.* The histograms with different letters are statistically different (P < 0.05). 1= mineral oil; 2= mineral oil + B. carinata meal; 3= vegetable oil; 4= vegetable oil + B. carinata meal.
- Figure 9 shows leaf tissue (% of control) of beet plants infected by mildew *Erysphe betae.* The histograms with different letters are statistically different (P < 0.05).
- Figure 10 shows mortality (in percentage) of *Myzus persicae* (Sulzer). The values were corrected with respect to the intreated control. The histograms with different letters are statistically different (P < 0.05). 1= vegetable oil; 2= vegetable oil + B. carinata meal; 3= vegetable oil + 10 g l-¹ of NaHCO₃.
- Figure 11 shows nortality (in percentage) of *Myzus persicae* (Sulzer). The values were corrected with respect to the untreated control. The histograms with different letters are statistically different (P < 0,05). 1= vegetable oil; 2= vegetable oil + B. carinata meal; 3= vegetable oil + 10 g l⁻¹ of NaHCO₃.
- Figure 12 shows survival (in percentage) of *Cacopsylla pyri.* The histograms with different letters are statistically different (P < 0.05).

More specifically, the meal is produced from Brassicaceae seeds and this meal usually has a glucosinolate content between 50 and 250 µmol per gram of seed. These thioglucosides present in nature in more than 120 variations, with little bioactivity on their own, are easily hydrolyzed in the presence of water and the endogenous enzyme myrosinase. On the other hand, the multiple products of hydrolytic decomposition, such as isothiocyanates, thiocyanates, nitrils, epithionitrils, thiones, etc., that can be obtained depending on the conditions of the enzymatic catalysis and of the precursor subjected to hydrolysis (glucosinolate), present a higher cytotoxic activity. Of these, the isothiocyanates are by far the most interesting molecules due to their intense cytotoxic activity.

Due to their chemical structure, dose and reference system, these molecules possess antitumoral and antimicrobial activities in higher organisms, as well as insecticide/insectifuge, nematicide, repellent and fungitoxic properties when used against soil and plant pathogen insects or fungi.

Mineral oils consist of paraffin oils obtained by petroleum refining. They have been used for many years in agriculture in 1-3% aqueous emulsions to combat some fruit plant and shrub insects. Some types of highly refined oils with a low level of phytotoxicity are also sometimes used on herbaceous evergreen plants.

They have different effects on insects. The main one is physical, since by obstructing the insect's respiratory tract, the oil causes its death by asphyxia, while in other cases they can have a toxic action by interacting, for example, with the fatty acids and the metabolism of the insect or by affecting the feeding methods of the insect. Vegetable oils, obtained by pressing or extraction with solvents from plant seeds, also have some application in agriculture, mainly as animal or insect repellents. Soya oil (Glycine max) and jojoba oil (Simmondsia chinensis) have a moderate degree of activity. It is thought that they produce a physical barrier between the insect and the plant. Sesame (Sesamum indicum), cotton (Abutilon theophrasti), neem (Azadirachta indica) and brassicaceae (Brassica napus, Brassica campestris) oils also contain molecules or complexes of molecules with biological activity that supplement the physical action of the oil.

The formulation according to the invention consists of a dispersion in meal oil (with a high quantitative content of specific glucosinolates) and subsequent emulsion in an aqueous solution when the treatment is carried out. An emulsifying agent can be added to the meal oil formulation to facilitate dispersion of the oil in the aqueous solution. The principle of activity of the composition is based on the combination between the asphyxiating effect of the oil and the fumigating effect of the toxic molecule released as a result of the hydration of the meal.

The oil that must incorporate the meal can be of mineral origin with a limited distillation range (160-250°C) or plant origin, preferably, but not exclusively, long chain (such as Crambe abyssinica, Brassica carinata oil, etc.). The meal dispersed in the oil must necessarily be obtained from a Brassicacea with a high glucosinolate content.

### Example no. 1

Assessment of the insecticide activity of the composition oil + Brassica carinata meal containing sinigrin, carried out on cochineal insects (Aonidiella aurantii) present on lemon fruits (Citrus limon). Aonidiella aurantii is a considerably polyphagous cochineal that attacks citrus fruits and other tree cultivations in particular.

The tests, carried out in the laboratory, concerned the formulation with mineral oil (1st test), the formulation with vegetable oil (2nd test), the dose effect check (3rd test) and the comparison with other meals containing different glucosinolates (4th test).

All the data collected were corrected according to the Abbott formula and subjected to analysis of variance(Fisher LSD method), using the SigmaPlot SPW7 version 2001 software.

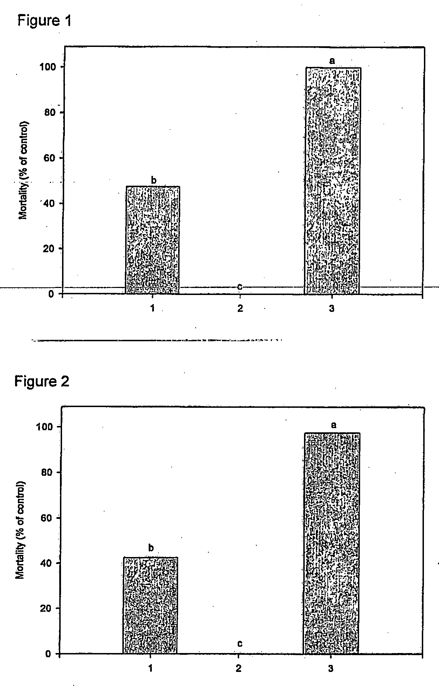

As far as the 1st test is concerned, the following results were obtained (Fig. 1):
- with just mineral oil (2%) in an aqueous solution, a 47.5% increase in mortality was achieved with respect to the untreated control;
- with just Brassica carinata meal (2g l⁻¹) in an aqueous solution, no increase in mortality was achieved with respect to the untreated control;
- with the composition mineral oil (2%) + meal with sinigrin (2g l⁻¹) in an aqueous solution, a 100% increase in mortality was achieved with respect to the untreated control.

The second test, replacing the mineral oil with vegetable oil, gave the following results (Fig. 2):
- with just vegetable oil (2%) in an aqueous solution, a 42.5% increase in mortality was achieved with respect to the untreated control;
- with just Brassica carinata meal (2g l⁻¹) in an aqueous solution, no increase in mortality was achieved with respect to the untreated control;
- with the composition vegetable oil (2%) + meal with sinigrin (2g l⁻¹) in an aqueous solution, a 97.5% increase in mortality was achieved with respect to the untreated control.

The third test, carried out to check the dose effect, showed that (Tab. 1):
- with the composition mineral oil (2%) + meal with sinigrin (0.75g l⁻¹) in an aqueous solution, a 90% increase in mortality (LD90) was achieved with respect to the untreated control.
- with the composition vegetable oil (2%) + meal with sinigrin (0.80g l⁻¹) in an aqueous solution, a 90% increase in mortality (LD90) was achieved with respect to the untreated control.
- with the composition mineral oil (2%) + meal with sinigrin (0.39g l⁻¹) in an aqueous solution, a 75% increase in mortality (LD75) was achieved with respect to the untreated control.
- with the composition vegetable oil (2%) + meal with sinigrin (0.47g l⁻¹) in an aqueous solution, a 75% increase in mortality (LD75) was achieved with respect to the untreated control.
- with the composition mineral oil (2%) + meal with sinigrin (0.04g l⁻¹) in an aqueous solution, a 60% increase in mortality (LD60) was achieved with respect to the untreated control.
- with the composition vegetable oil (2%) + meal with sinigrin (0.13g l⁻¹) in an aqueous solution, a 60% increase in mortality (LD60) was achieved with respect to the untreated controd.

Table 1 - Lethal Dose (LD) of 60, 75 and 90% expressed in grams of meal containing sinigrin + (2g of oil)/litre of aqueous solution.

| Treatments | LD60 | LD75 | LD90 | Estimated Standard Error |
|---|---|---|---|---|
| | g | g | g | g |
| Min. oil + meal with sinigrin | 0.04 | 0.39 | 0.75 | +/-0.20 |
| Veg. oil + meal with sinigrin | 0.13 | 0.47 | 0.80 | +/-0.19 |

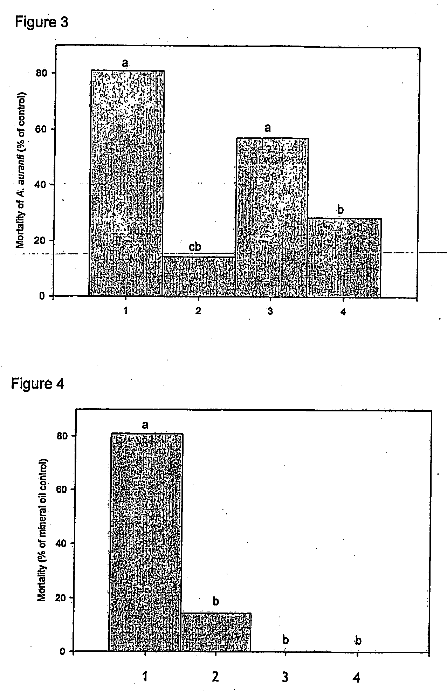

The fourth test, carried out to check the efficacy of the glucosinolate sinigrin with respect to other types of glucosinolates, gave the following results (Fig. 3):
- with the composition mineral oil (2%) + Brassica carinata meal containing sinigrin (0.66g l⁻¹) in an aqueous solution, an 80.97% increase in mortality was achieved with respect to the use of just mineral oil;
- with the composition mineral oil (2%) + sunflower meal (0.66g l⁻¹) in an aqueous solution, a non-significant increase in mortality was achieved with respect to the use of just mineral oil;
- with the composition mineral oil (2%) + Brassica verna meal containing nasturtin (0.66g l⁻¹) in an aqueous solution, a 57.15% increase in mortality was achieved with respect to the use of just mineral oil;
- with the composition mineral oil (2%) + Eruca sativa meal containing erucin (0.66g l⁻¹) in an aqueous solution, a 28.60% increase in mortality was achieved with respect to the use of just mineral oil.

The following conclusions can be drawn from these results:
- the insecticide effect can be attributed to the combined action of the meal containing sinigrin and mineral and/or vegetable oil, and the combined action of the two systems is clearly superior to that of the individual systems. In fact, when the meal with sinigrin is dispersed directly in the aqueous solution, i.e. without the mineral and/or vegetable oil, it has no insecticide effect (Figs. 1 and 2).
- the insecticide effect of the meal with sinigrin is greater than that of other meals containing other types of glucosinolates (Fig. 3).
- by varying the quantity of meal with sinigrin dispersed in mineral and/or vegetable oil, we found a clear "dose effect" (Tab. 1).

### Example no. 2

The activity of the composition described in Example 1 was assessed on epiphytic habitat pathogen fungi. The tests were carried out on sugar beet oidium (Erysiphe polygoni) using the standard dose of meal with sinigrin (2g l⁻¹) and 1.5% mineral oil instead of 2%. The assessments concerned both the ability to prevent the colonization of the pathogen on the host and the degree of limitation of the disease when infection has already occurred.

In this case too, the results show that the composition "aqueous solution + mineral oil + meal with sinigrin" has a significantly superior activity with respect to the emulsion of just oil or to the suspension of just meal.

The doses of the composition components are as follows:
- mineral or vegetable oil with a minimum dosage of the aqueous solution;
- emulsifying agent to be added to the vegetable oil;
- Brassicaceae meal (containing from 50 to 250 µmol per gram of glucosinolate seeds) with a minimum dosage of 0.04g l⁻¹.

This composition, prepared just before it is to be used, must be emulsified with an aqueous solution of other compounds (salts, organic solvents, etc.) present in varying percentages.

According to an approximate calculation, to achieve a 60% reduction of the presence of the red cochineal (Aonidiella auranti) infesting a hectare of orange grove requires approximately 78g of meal with sinigrin, mixed in 12 litres of vegetable oil (including the emulsifying agent).

Meals containing sinigrin, with respect to those containing other glucosinolates, are more effective since the allyl-isothiocyanate which is produced as a result of the hydrolytic catalysis of sinigrin is more volatile due to the lower molecular weight of the molecule.

It must be pointed out that the overall effect of the composition is unexpectedly much greater than the sum of the separate effects of the oil, or other modulator means, and the pure Brassicaceae seed meal or mixed with water.

The composition also provides a prolonged effect over time, with an intensity and duration that can easily be adjusted by varying the percentages of the components or by an appropriate choice of the components.

### Example no. 3

### Tests on citrus fruit red scale insect (Aonidiella aurantii)

### Laboratory tests

The mineral oil based composition was used on third generation red scale insects (Aonidiella aurantii), present on ripe oranges picked from untreated plants from the production area of Floridia, Sicily. The composition consisted of 0.66g l⁻¹ of meal dispersed in UF mineral oil used at 2% of the water.

The following were compared: Brassica carinata meal containing sinigrin, Helianthus annuus meal with no glucosinolate content and deactivated B. carinata meal (following hydration and subsequent drying of the meal in order to hydrolyze all the sinigrin).

The formulation was applied with an atomizer, carrying out two treatments with an interval of 7 days between them. achieving a mortality of 200 insects for each meal tested (50 per repetition). The insect was considered dead when, raising the "scutellum" with a loop, it had lost turgidity.

All the data collected were corrected according to the formula of (Abbott, 1925) and subjected to the analysis of variance (Fisher LSD method), using the SigmaPlot SPW7 version 2001 software.

The corrected mortality values show that, of the various Brassica meals used, the B. carinata meal containing sinigrin was the most effective one (Fig. 4).

The B. carinata meal containing sinigrin increased the mortality by 80.9% with respect to the control, while the H. annuus meal, containing no glucosinolates, increased mortality by only 14.3%. The insecticide effect of the meal is therefor correlated with the release of isothiocyanate. In fact, using deactivated meal (the hydrolysis reaction was triggered before the test, removing the isothiocyanates produced) in mineral oil did not lead to any increase in mortality with respect to just oil (Fig. 4). The treatment with B. carinata meal dispersed directly in water without oil also showed no biocidal effect, confirming the fact that the dispersion in oil and formation of an emulsion are essential in exalting the insecticide efficacy of sinigrin. The efficacy of this composition in controlling citrus fruit red scale insect could be due to the fact that, when emulsified, the grains of meal dispersed in the oil rapidly release almost all their sinigrin content into the water and only after several hours, i.e. when the product has been applied on the plants, does the isothiocyanate start to be released, the majority of which is immediately transformed into an oily solution and then comes into contact with the insect. The asphyxiating effect of the oil is thus supplemented by the toxic action of the products of glucosinolate hydrolysis. In addition, thanks to the oil the grains of meal adhere to the surface of the plant tissue and to the insects. It is presumed that, by forming a sort of film around the plant tissue and on the surface of the grains of meal, the oil improves the contact of the isothiocyanate (contained within the meal) with the parasite.

### Example no. 4

### Tests on citrus fruit red scale insect (Aonidiella aurontii)

### Laboratory tests

The present invention is based on the combination of a carrier (e.g. oil) with Brassicacee meal.

Unlike previous inventions (Patent no. US6,545,043 B1), in which chemical compounds derived from the hydrolysis of glucosinolates are transported, in this invention the carrier transports a non-active complex (meal), containing glucosinolate and myrosinase which are only activated in certain circumstances, releasing their active ingredients and producing greater efficacy with respect to the pure product.

The novelty of this invention lies in the activation of the preparation on contact with the target and in the modulation of the release of toxic molecules. The study of the water-oil-meal three-phase reaction made it possible to identify the dynamics of the release over time of their precursors (glucosinolates).

Laboratory studies were therefore carried out in which, using the "composition" prepared in a preliminary phase (2 g l⁻¹ of B. carinata meal + 2% vegetable oil in water), the release of the biocide active ingredients over time was assessed. This activity included a study of the dynamics of glucosinolates, assessing the level over time in the water phase and in the meal phase.

The results, reported summarily in Tab. 2, show that the glucosinolates are rapidly solubilised in water, in a period that can indicatively be in the region of 20 minutes, similar to the soluble amount of myrosinase present in the meal.

**Tab. 2 - Assessment over time of the level of glucosinolates in the water phase (0.2 g meal 2 ml vegetable oil 100 ml water) with meals with varying content of myrosinase.**

| Composition (2ml oil+0.2 g meal) in 100 ml of water | Theoretical GLS | Glucosinolates (µmol in flask) | | | Glucosinolates (yield %) | | |
|---|---|---|---|---|---|---|---|
| | µmol in flask | Water | Meal | Total | Water | Meal | Total |
| 0 minutes | 30 | | | | | | |
| 20 minutes | | 29.7 | 1.1 | 30.8 | 98,5 | 1.0 | 99.5 |
| 30 minutes | | 30.0 | 0.7 | 30.7 | 99 | 0.7 | 99.7 |
| 3 hours | | 28.8 | traces | 28.8 | 96 | traces | 96 |
| 5 hours | | 14.4 | traces | 14.4 | 14.4 | traces | 48.0 |

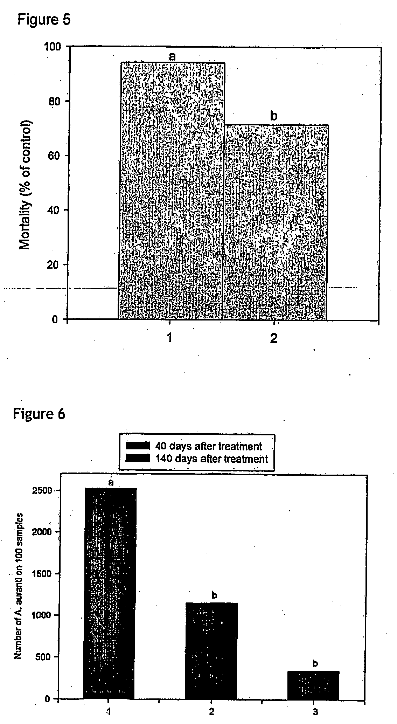

The composition with B. carinata meal containing sinigrin, characterised by a low myrosinase content, solubilises the glucosinolate in water very rapidly, maintaining it unchanged for several hours and only halving it after more than five hours following its degradation into isothiocyanate (Tab. 2) The scale-infested oranges used in the previous test were treated with three different compositions: vegetable oil + B. carinata meal; vegetable oil + isothiocyanate (synthesis); control (treated with water). The vegetable oil was appropriately emulsified at 2% of the water, while the meal was applied at 2 g l⁻¹ of water. The quantity of isothiocyanate added to the oil was the same as the meal content.
The results (Fig.5) show that the B. carinata meal was again the most efficacious with 94.3% mortality, while in the test with synthesis isothiocyanate the mortality was 71%.

These data confirm the importance of the grains of meal on the surface of the plant tissue and the insect, as they allow a modulated releas of the active ingredients over time. In fact, the insecticide action which is obtained when synthesis isothiocyanate or other products of glucosinolate hydrolysis are dissolved in oil is significantly lower than that obtained with the presence of B. carinata meal. The presence of other molecules (in addition to those deriving from the hydrolysis of the glucosinolate) could be the reason for this greater efficacy. It could also be due to the fact that the contact of the grains of meal with the treated surface produces a slight phytotoxicity (reddish colouring of the fruit or bronzing on the leaves of greenhouse-grown melons) which makes the plant more resistant to the pathogen.

### Example no. 5

### Tests on citrus fruit red scale insect (Aonidiella aurantii)

### Field test

The composition was tested in the control of red scale insects on an orange grove located in Floridia, Sicily. Three treatments were tested on 20 plants each: untreated control, mineral oil + B. carinata meal, vegetable oil (with 8% of the emulsifying agent ethilon OL6) + B. carinata meal. The oils were used at 1.5% of the water.
Two samplings were carried out 40 and 140 days after the treatment; 100 fruits were collected for each treatment and the red scale insects on each fruit were counted.
The results obtained confirmed the laboratory data, albeit in the presence of a low level of infestation recorded over the entire year.
Figure 6 shows a reduction of the insects (compared to the control) for the two treated groups 40 days after treatment.

At the time of harvest, i.e. 140 days after treatment, the untreated oranges also showed a significant difference with respect to the treated ones. In particular, the oranges treated with the vegetable oil based composition presented a much lower number of scale insects than the control (Fig: 6).
All the data collected were corrected according to the Abbott formula and subjected to analysis of variance(Fisher LSD method), using the SigmaPlot SPW7 version 2001 software.

### Example no. 6

### Test on European red mite Panonychus ulmi infesting sugar beet

### Laboratory tests

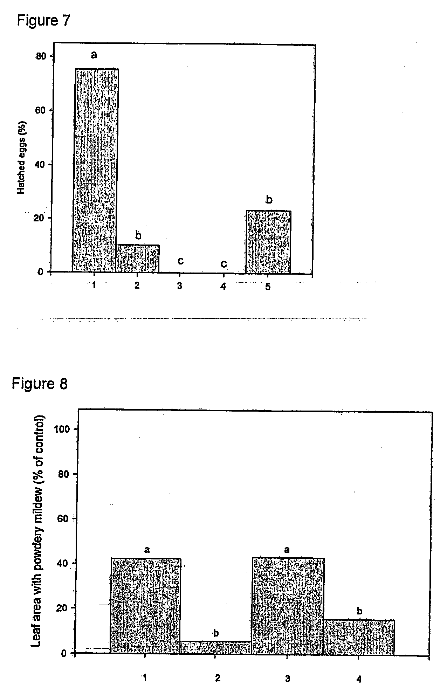

Beet leaf discs measuring 60 mm were placed in a 90 mm diameter Petri dish and kept in a climatic cell at 24°C, 75% R.H. with 17 hours of light. Fifteen female mites were placed on each leaf disc to lay their eggs. The females were removed after 24 hours and the eggs were counted. Each group consisted of three leaf discs. The treatments were carried out with the "deep test" method: the leaf discs were immersed for five seconds in a suspension consisting of 1.5% vegetable oil, 1.5% vegetable oil + 2 g l⁻¹ carinata meal, 1.5% UF mineral oil + 2 g l⁻¹ carinata meal or the acaricide hexythiazox Matacar (Sipcam) at a dose of 50mg p.a. l⁻¹. The control was immersed in water. The ovicide activity was assessed starting on the fourth day and every 2 days up to the eighth day, calculating the percentage of hatched eggs estimated indirectly by counting the live offspring. Figure 7 shows that no eggs had hatched after 8 days in the groups treated with the vegetable formulations.

Only 10% of the eggs treated with just crambe vegetable oil hatched, while with the commercial acaricide 23.4% of the eggs hatched and with water 75.4% hatched after 8 days (Fig. 7).
In addition to the reasons described above, the efficacy of the formulation used against European red mite eggs may also be due to the fact that the grains of meal, dispersed when the treatment is applied, are trapped by the web which "anchors" and protects the eggs laid by the mite, thus probably concentrating the release of isothiocyanate on this web.
The female mite tends, in fact, to lay her eggs in groups and attach them to the plant tissue by means of a thick web. The closeness of the grains of meal to the eggs very probably creates a toxic environment, thereby interfering with their normal development.

### Example no. 7

### Test on sugar beet mildew Erysiphe betae (Vanha)

### Laboratory test

Beet plants with a high level of infestation of mildew were raised in the greenhouse at a temperature of 24°C and humidity of approximately 60%.

A 1.5% emulsion of oil in water with a meal content of 2g l⁻¹ of water was prepared with the oil and meal compounds.

Five groups were assessed with 3 repetitions, with randomized lots of 4 plants. In the first group the plants were treated with water (control), in the second with mineral oil, in the third with mineral oil + B. carinata meal, in the fourth with vegetable oil and in the fifth with vegetable oil + B. carinata meal. The oils were used at 1.5% of the water. Two treatments were carried out (using a manual atomizer) with an interval of 10 days between them. To assess the extent of the surface affected by the mildew, the plants were attributed a value of 1 to 5 (1=0-20%; 2=21-40%; 3=41-60%; 4=61-80%; 5>80%). Significant differences between the groups were observed (Fig. 8).

The groups treated with just oil showed a 42% infection (with respect to the control), while in those treated with mineral oil + meal and vegetable oil + meal the percentages fell to 5.6% and 15.7% respectively.

### Example no. 8

### Tests on cucurbit mildew Erysiphe cichoracearum DC

### Field tests

The tests were carried out on melons (Cucumis melo), grown in a tunnel, 30 days after harvesting, in Sermide, northern Italy, with an experimental design of 3 groups and 3 repetitions, and plots in randomized blocks of 8 plants. An inspection was performed the week before the test in order to quantify the degree of infection, which proved to be widespread despite regular treatment with sulphur-based products. Two treatments were carried out with an interval of 10 days between them, using a shoulder-carried sprayer. Ten days after the second treatment, two leaves per plant were collected and the percentage of affected upper and lower surface was assessed.

The data were subjected to analysis of variance (Fisher LSD method), using the "SigmaPlot SPW7 version 2001" software.

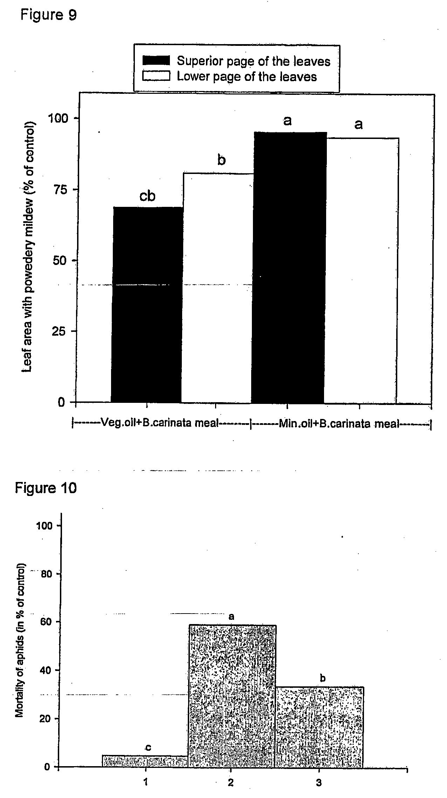

The results of this test show a lower presence of infection in the treated groups with respect to the untreated control (Fig. 9).

In the group treated with vegetable oil + B. carinata meal, the leaf area with mildew decreased by 31% with respect to the control on the upper surface and 19% on the lower surface.

With regard to the group treated with mineral oil + B. carinata meal, the leaf area with mildew decreased by 4.4% on the upper surface and 9.6% on the lower surface (data not statistically significant).

These data confirm the results obtained in the greenhouse for the control of beet mildew (although with a different species of mildew). The efficacy shown by the combination oil + B. carinata meal for field control of cucurbit mildew Erysiphe cichoracearum offers excellent application prospects also for the control of this widespread pathogen fungus.

The field tests were carried out when the production cycle was almost complete (about 30 days before harvesting) and, at the time of the first treatment, considerable amounts of sulphur were present from previous treatments. This massive presence of sulphur could be the reason for the lesser efficacy of the combination mineral oil + B. carinata meal with respect to vegetable oil + B. carinata meal.

In addition, in the plots treated with mineral oil + B. carinata meal the leaves presented widespread necrotic spots; phytotoxicity due to the contact between mineral oil and sulphur. The lower degree of phytotoxicity (just bronzing) found on the plots treated with vegetable oil + B. carinata meal suggests that the composition may not be phytotoxic in the presence of sulphur and may even be reinforced with the addition of limited doses of mineral sulphur. In this case, the cytotoxic action of the formulation could be in synergy with the anti-mildew action of the sulphur.

All the data collected were subjected to analysis of variance (Fisher LSD method), using the "SigmaPlot SPW7 version 2001" software.

### Example no. 9

### Tests on beet aphids Aphis fabae (Scopoli)

### Laboratory test

Beet plants with a high level of infestation of Myzus persicae (Sulzer) were grown in the greenhouse at a temperature of 24°C and humidity of around 60%. Four groups were compared, with 3 repetitions and randomized lots of 3 plants: 1.5% vegetable oil, vegetable oil + B. carinata meal (2 g l⁻¹), 0.5% vegetable oil + 10 g l⁻¹ of NaHCO₃, control treated with H₂O.

Just one treatment was carried out (using a manual sprayer), and after 24h the efficacy of the treatment was assessed by counting the number of live and dead aphids on one leaf.

The results show that formulation presents a mortality of 60% with respect to the control versus 5% in the group treated with just oil and 30% in the group treated with oil and bicarbonate of soda (Fig. 10).

### Example no. 10

### Tests on Myzus persicae (Sulzer)

### Field tests

Tests were carried out on plants of Cirsium arvense, infested by Aphis fabae. An experimental design was performed with 4 groups and 3 repetitions, with 3 plants per repetition: 1.5% vegetable oil; vegetable oil + meal (2 g l⁻¹); 0.5% vegetable oil + 10 g l⁻¹ of NaHCO₃; control treated with H₂O.

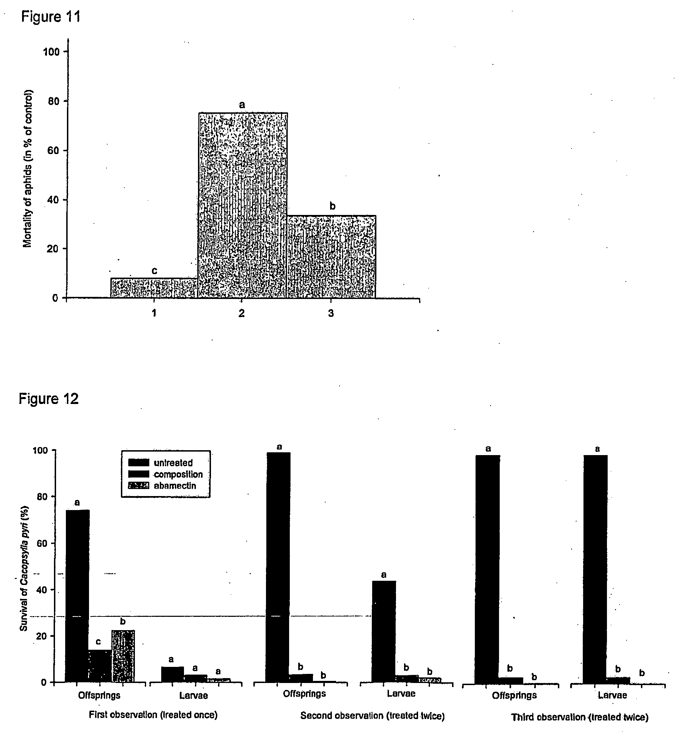

Twenty-four hours after the treatment 2 leaves were taken from each plant: one from the base of the plant and one from the mid-upper part. As can be seen in figure 11, the mortality of the aphids was very similar (only slightly higher) than the level obtained in the controlled environment test.

### Example no. 11

### Test on Cacopsylla pyri

### Field tests

Pear plants with a high level of lice infestation were treated on May 6 and 20 2006. An experimental design was performed with 3 groups and 4 repetitions, with 7 plants per repetition: 1.5% vegetable oil; vegetable oil + B. carinata meal (composition); Abamectin (Vertimec) at the recommended dose; untreated control

The results were assessed ten days after the treatment, counting the total number of offspring and nymphs on each bud. The results shown in figure 12 demonstrate that the composition can control the development of the lice very well, even in comparison with the best product currently available on the market, Abamectin (Vertimec).

The data collected during the first treatment show a greater efficacy of the formulation with respect to the synthesis product. Ten days after the first treatment, there were 74, 14 and 22 surviving offspring for the control, the composition and abamectin, respectively. Ten days after the second treatment, the presence of offspring and nymphs (larvae stages) in the treated groups was insignificant, while in the untreated control the presence of offspring was 100% and over 40% of nymphs. At the third observation, no offspring or nymphs were found in the group treated with abamectin, only 2 or 3% in the group treated with vegetable oil + B. carinata meal and 100% in the control group. This could be explained by the fact that the active synthesis ingredient (abamectin) has a prolonged persistence over time (in fact, no insects were found 30 days after the second treatment), while in the group treated with vegetable oil + B. carinata meal, although the lice population was well controlled (only 2-3%) the composition did not produce the "sterilizing" effect found in the synthesis product group. This must all be considered as positive since modern agriculture has replaced the concept of "parasite destruction" with the softer concept of "population control". Vegetable oil + B. carinata meal, controlling the lice without "sterilizing" them, alters the extremely complex relationship between endoparasites and their victims to a lesser extent. This is extremely important since useful insects are often able to limit plant-eating populations within "almost equilibrium" limits".
One advantage of this invention is to provide a composition with a high degree of efficacy against numerous types of infestants and infectants, such as fungi, bacteria, insects and other biological agents that can attack plants, wooden objects or other organic elements.
Another advantage is to provide a composition that is practically harmless for humans and pets, easy to package and transport and which has a low environmental impact.

## Claims

1. A composition for the treatment and/or prevention of attacks by biological agents on plants **characterized in that** it comprises at least:
- a modulator means comprising at least one oil or fat of vegetable, animal, mineral or synthetic origin;
- a by-product of Brassicaceae seeds comprising a meal of Brassicaceae seeds having a glucosinate content between 50 and 250 µmol; and
- water;
the Brassicaceae seed by-product, comprising the meal of Brassicaceae seeds, is mixed with the modulator means, comprising the oil or fat, to form an emulsified mixture in the water.

2. A composition according to claim 1, **characterized in that** the modulator means comprises at least one gelatine or gelatinizing agent.

3. A composition according to claim 1 or 2, **characterized in that** the modulator means comprises at least one thickening agent.

4. A composition according to any of the preceding claims, **characterized in that** the seeds are Brassica carinata, Brassica verna or Eruca sativa.

5. A composition according to claim 1, **characterized in that** the percentage in weight of the modulator means is at least 0.1 %.

6. A composition according to claim 1 or claim 5, **characterized in that** the percentage in weight of the by-product of Brassicaceae seeds is at least 0.0004%.

7. A composition according to claim 1, 5 or 6, **characterized in that** the percentage in weight of the water is at least 1.0%.

8. A composition according to any of the preceding claims, **characterized in that** it also comprises at least one emulsifying agent, dispersing agent or thickening agent.

9. A composition according to any of the preceding claims, **characterized in that** the water is in pure form or in an aqueous solution.

10. A method for producing the composition according to any of the preceding claims **characterized in that**:
- the Brassicaceae seed by-product, which comprises a meal of Brassicaceae seeds having a glucosinate content between 50 and 250 µmol, is mixed with the modulator means, which comprises at least one oil or fat of vegetable, animal, mineral or synthetic origin, to obtain a mixture;
- the mixture is packaged;
- before the mixture is used, it is emulsified with water in predetermined percentages.

11. Use of the composition according to any of the preceding claims from 1 to 9 as an insecticide for plant infestants, as a cytotoxic agent for plant pathogens, as a fungitoxic agent for plants, as an antibacterial agent for plants.

12. Use of the composition according to any of the preceding claims from 1 to 9 as an insecticide for plants wherein; the composition further comprises glucosidase enzymes and thioglucosidase enzymes.

13. Use of the composition according to any of the preceding claims from 1 to 9 as a cytotoxic agent for plant pathogens; wherein the composition further comprises glucosidase enzymes and thioglucosidase enzymes.

14. Use of the composition according to any of the preceding claims from 1 to 9 as a fungitoxic agent for fungus pathogens for plants; wherein the composition further comprises glucosidase enzymes and thioglucosidase enzymes.

## Patentansprüche

1. Zusammensetzung zur Behandlung und/oder Prävention von Angriffen durch biologische Agentien auf Pflanzen, **dadurch gekennzeichnet, dass** sie wenigstens Folgendes umfasst:
- ein Modulatormittel, das wenigstens ein Öl oder Fett pflanzlichen, tierischen, mineralischen oder synthetischen Ursprungs umfasst;
- ein Nebenprodukt von Brassicaceae-Samen, das einen Schrot von Brassicaceae-Samen mit einem Glucosinat-Gehalt zwischen 50 und 250 µmol umfasst; und
- Wasser;
wobei das Nebenprodukt von Brassicaceae-Samen, das den Schrot von Brassicaceae-Samen umfasst, mit dem Modulatormittel, das das Öl oder Fett umfasst, gemischt wird, wobei ein emulgiertes Gemisch in dem Wasser entsteht.

2. Zusammensetzung gemäß Anspruch 1, **dadurch gekennzeichnet, dass** das Modulatormittel wenigstens eine Gelatine oder wenigstens ein Gelatinisierungsmittel umfasst.

3. Zusammensetzung gemäß Anspruch 1 oder 2, **dadurch gekennzeichnet, dass** das Modulatormittel wenigstens ein Verdickungsmittel umfasst.

4. Zusammensetzung gemäß einem der vorstehenden Ansprüche, **dadurch gekennzeichnet, dass** es sich bei den Samen um *Brassica carinata, Brassica verna* oder *Eruca sativa* handelt.

5. Zusammensetzung gemäß Anspruch 1, **dadurch gekennzeichnet, dass** der prozentuale Gewichtsanteil des Modulatormittels wenigstens 0,1% beträgt.

6. Zusammensetzung gemäß Anspruch 1 oder 5, **dadurch gekennzeichnet, dass** der prozentuale Gewichtsanteil des Nebenprodukts der Brassicaceae-Samen wenigstens 0,0004% beträgt.

7. Zusammensetzung gemäß Anspruch 1, 5 oder 6, **dadurch gekennzeichnet, dass** der prozentuale Gewichtsanteil des Wassers wenigstens 1,0% beträgt.

8. Zusammensetzung gemäß einem der vorstehenden Ansprüche, **dadurch gekennzeichnet, dass** sie auch wenigstens einen Emulgator, Dispersionsmittel oder Verdickungsmittel umfasst.

9. Zusammensetzung gemäß einem der vorstehenden Ansprüche, **dadurch gekennzeichnet, dass** das Wasser in reiner Form oder als wässrige Lösung vorliegt.

10. Verfahren zur Herstellung der Zusammensetzung gemäß einem der vorstehenden Ansprüche, **dadurch gekennzeichnet, dass**:
- das Nebenprodukt von Brassicaceae-Samen, das einen Schrot von Brassicaceae-Samen mit einem Glucosinat-Gehalt zwischen 50 und 250 µmol umfasst, mit dem Modulatormittel, das wenigstens ein Öl oder Fett pflanzlichen, tierischen, mineralischen oder synthetischen Ursprungs umfasst, gemischt wird, wobei man ein Gemisch erhält;
- das Gemisch verpackt wird;
- das Gemisch, bevor es verwendet wird, mit Wasser in vorbestimmten Prozentanteilen emulgiert wird.

11. Verwendung der Zusammensetzung gemäß einem der vorstehenden Ansprüche 1 bis 9 als Insektizid für Pflanzenschädlinge, als cytotoxisches Mittel für Pflanzenpathogene, als fungitoxisches Mittel für Pflanzen, als antibakterielles Mittel für Pflanzen.

12. Verwendung der Zusammensetzung gemäß einem der vorstehenden Ansprüche 1 bis 9 als Insektizid für Pflanzen, wobei die Zusammensetzung weiterhin Glucosidase-Enzyme und Thioglucosidase-Enzyme umfasst.

13. Verwendung der Zusammensetzung gemäß einem der vorstehenden Ansprüche 1 bis 9 als cytotoxisches Mittel für Pflanzenpathogene, wobei die Zusammensetzung weiterhin Glucosidase-Enzyme und Thioglucosidase-Enzyme umfasst.

14. Verwendung der Zusammensetzung gemäß einem der vorstehenden Ansprüche 1 bis 9 als fungitoxisches Mittel für pathogene Pilze für Pflanzen, wobei die Zusammensetzung weiterhin Glucosidase-Enzyme und Thioglucosidase-Enzyme umfasst.

## Revendications

1. Composition pour le traitement et/ou la prévention d'attaques par des agents biologiques sur des plantes **caractérisée en ce qu'**elle comprend au moins :
- un moyen modulateur comprenant au moins une huile ou une graisse d'origine végétale, animale, minérale ou synthétique ;
- un sous-produit de semences de Brassicaceae comprenant une farine de semences de Brassicaceae ayant une teneur en glucosinate comprise entre 50 et 250 µmol ; et
- de l'eau ;
le sous-produit des semences de Brassicaceae, comprenant la farine de semences de Brassicaceae, étant mélangé au moyen modulateur, comprenant ladite huile ou graisse, afin de former un mélange émulsifié dans l'eau.

2. Composition selon la revendication 1, **caractérisée en ce que** le moyen modulateur comprend au moins une gélatine ou un agent gélifiant.

3. Composition selon la revendication 1 ou 2, **caractérisée en ce que** le moyen modulateur comprend au moins un agent épaississant.

4. Composition selon l'une quelconque des revendications précédentes, **caractérisée en ce que** les semences sont des semences de Brassica carinata, de Brassica verna ou d'Eruca sativa.

5. Composition selon la revendication 1, **caractérisée en ce que** le pourcentage en poids du moyen modulateur est au moins égal à 0,1 %.

6. Composition selon la revendication 1 ou la revendication 5, **caractérisée en ce que** le pourcentage en poids du sous-produit de semences de Brassicaceae est au moins égal à 0,0004 %.

7. Composition selon la revendication 1, 5 ou 6, **caractérisée en ce que** le pourcentage en poids d'eau est au moins égal à 1,0 %.

8. Composition selon l'une quelconque des revendications précédentes, **caractérisée en ce qu'**elle comprend également au moins un agent émulsifiant, un agent dispersant ou un agent épaississant.

9. Composition selon l'une quelconque des revendications précédentes, **caractérisée en ce que** l'eau est sous forme pure ou d'une solution aqueuse.

10. Procédé de production de la composition selon l'une quelconque des revendications précédentes **caractérisé en ce que** :
- le sous-produit des semences de Brassicaceae, qui comprend une farine de semences de Brassicaceae ayant une teneur en glucosinate comprise entre 50 et 250 µmol, est mélangé au moyen modulateur, qui comprend au moins une huile ou une graisse d'origine végétale, animale, minérale ou synthétique, afin d'obtenir un mélange ;
- le mélange est conditionné ;
- avant que le mélange ne soit utilisé, celui-ci est émulsifié avec de l'eau dans des pourcentages prédéfinis.

11. Utilisation de la composition selon l'une quelconque des revendications 1 à 9 précédentes, comme insecticide contre des parasites de végétaux, comme agent cytotoxique contre des pathogènes végétaux, comme agent fongitoxique pour les plantes, comme agent antibactérien pour les plantes.

12. Utilisation de la composition selon l'une quelconque des revendications 1 à 9 précédentes, comme insecticide pour les plantes ; dans laquelle la composition comprend en outre des enzymes glucosidase et des enzymes thioglucosidase.

13. Utilisation de la composition selon l'une quelconque des revendications 1 à 9 précédentes, comme agent cytotoxique contre des pathogènes végétaux ; dans laquelle la composition comprend en outre des enzymes glucosidase et des enzymes thioglucosidase.

14. Utilisation de la composition selon l'une quelconque des revendications 1 à 9 précédentes, comme agent fongitoxique contre des pathogènes fongiques pour les plantes ; dans laquelle la composition comprend en outre des enzymes glucosidase et des enzymes thioglucosidase.
